# EUROPEAN PATENT APPLICATION

(11) **EP 4 325 508 A1**
(43) Date of publication of application: **21.02.2024**
(21) Application number: 22190697.7
(22) Date of filing: 17.08.2022
(51) Int. Cl.: G16H 10/60

(54) **COMPUTER-IMPLEMENTED METHOD FOR TRANSFERRING DATA AND MEDICAL DATA SYSTEM**

(71) Applicant: BIOTRONIK SE & Co. KG, 12359 Berlin (DE)
(72) Inventor: Krüger, Daniel, 15741 Bestensee (DE); Müller, Jens, 14195 Berlin (DE); Deutschmann, Hendrik, 10247 Berlin (DE)
(74) Representative: Biotronik Corporate Services SE

(57) **Abstract**

The invention relates to a computer-implemented method for transferring data (D), in particular clinical and/or administrative data, from a first information system (12) of an IT service provider to a second information system (14) of a healthcare provider, in particular a hospital, via a data transfer protocol (P) used by the healthcare provider and having a data format (DF) used by the healthcare provider, comprising converting (S3) the data of the first information system (12) of an IT service provider, said data having a first data format (DF1), into data having a second data format (DF2) used by the healthcare provider, and transferring (S4) the converted data having the second data format (DF2) via the configured data transfer protocol (P) to the second information system (14) of the healthcare provider. The invention further relates to a medical data system (1).

## Description

The invention relates to a computer-implemented method for transferring data, in particular clinical and/or administrative data, from a first information system of an IT service provider to a second information system of a healthcare provider, in particular a hospital, via a data transfer protocol used by the healthcare provider and having a data format used by the healthcare provider.

Furthermore, the invention relates to a medical data system for transferring data, in particular clinical and/or administrative data, to a further medical data system of a healthcare provider, in particular a hospital, via a data transfer protocol used by the healthcare provider and having a data format used by the healthcare provider.

A hospital medical data infrastructure conventionally comprises a dedicated server configured to receive medical data, in particular from an electronic health record of an IT service provider. Said electronic health record may comprise medical data recorded by e.g. implantable medical devices which are configured to transfer recorded data via an internet connection to the electronic health record of the IT service provider.

The dedicated server of the hospital medical data infrastructure is further configured to convert the received data into another data format that is compatible with the hospital medical data infrastructure. This solution is however costly to maintain and operate as said server has to be configured and dedicated software to perform the intended functions needs to be developed.

It is therefore an object of the present invention to provide an improved method of transferring medical data from a first information system of an IT service provider to a second information system of a healthcare provider in a more efficient and user-friendly manner.

The object is solved by a computer-implemented method for transferring data, in particular clinical and/or administrative data, from a first information system of an IT service provider to a second information system of a healthcare provider, in particular a hospital, via a data transfer protocol used by the healthcare provider and having a data format used by the healthcare provider having the features of claim 1.

Alternatively or additionally, the first information system may receive data from the second information system, e.g. to store/process/interpret the data.

In addition, the object is solved by a medical data system for transferring data, in particular clinical and/or administrative data, to a further medical data system of a healthcare provider, in particular a hospital, via a data transfer protocol used by the healthcare provider and having a data format used by the healthcare provider having the features of claim 11.

Further developments and advantageous embodiments are defined in the dependent claims.

The present invention provides a computer-implemented method for transferring data, in particular clinical and/or administrative data, from a first information system of an IT service provider to a second information system of a healthcare provider, in particular a hospital, via a data transfer protocol used by the healthcare provider and having a data format used by the healthcare provider.

The method comprises accessing a web server of the first information system of the IT service provider by means of a client application, in particular of the healthcare provider, via an internet connection. The method further provides configuring a data transfer protocol and a data format for the data transfer between the first information system of an IT service provider and the second information system of a healthcare provider on the web server by means of the client application, wherein the configured data transfer protocol and data format correspond to the data transfer protocol and data format used by the healthcare provider.

The method moreover provides converting the data of the first information system of an IT service provider, said data having a first data format, into data having a second data format used by the healthcare provider. In addition, the method provides transferring the converted data having the second data format via the configured data transfer protocol to the second information system of the healthcare provider.

The present invention further provides a medical data system for transferring data, in particular clinical and/or administrative data, to a further medical data system of a healthcare provider, in particular a hospital, via a data transfer protocol used by the healthcare provider and having a data format used by the healthcare provider.

The system provides a web server and a client application for accessing the web server via an internet connection, wherein the client application is adapted to configure a data transfer protocol and a data format for the data transfer between the medical data system, in particular of an IT service provider, and the further medical system of the healthcare provider on the web server, wherein the configured data transfer protocol and data format correspond to the data transfer protocol and data format used by the healthcare provider.

Furthermore, the system provides a converting means configured to convert data of the medical data system, said data having a first data format, into data having a second data format used by the healthcare provider. In addition, the system provides data transfer means configured to transfer the converted data having the second data format via the configured data transfer protocol to the further medical data system of the healthcare provider.

The present invention moreover provides a computer program with program code to perform the method of the invention when the computer program is executed on a computer.

In addition, the present invention provides a computer-readable data carrier containing program code of a computer program for performing the method of the invention when the computer program is executed on a computer.

It is an idea of the present invention to provide a system and method to ease interoperability of a first information system of an IT service provider and a second information system of a healthcare provider. The healthcare provider, in particular the hospital, is thus able to configure via the client application data format and data transfer protocol of the data to be exported.

According to an aspect of the invention, the client application is configured to import a data definition and/or a sample data set from the second information system of the healthcare provider in a structured machine-readable format, in particular JSON, XML, CVS and/or JAML. With such a scheme it is advantageously possible to fully automize the configuration process. It is further possible to create a configuration file from the data definition as well as from a data set.

A data definition is understood to mean a data format, e.g. a text format and/or character length of a given data set.

According to a further aspect of the invention, a parsing algorithm is configured to parse the imported data definition and/or sample data set and suggest a data format and/or a data mapping between the first information system of the IT service provider and the second information system of the healthcare provider. The parser thus derives the configuration data from the content or structure of the data set.

According to a further aspect of the invention, the data of the first information system of the IT service provider having a first data format is converted into the second data format used by the healthcare provider according to the configuration made by the client application on the web server of the first information system. The user of the client application in the hospital IT environment is thus able to advantageously configure that the data is sent from the IT service provider in a data format compatible with the data format used in the hospital IT environment.

According to a further aspect of the invention, the client application is configured to provide a configuration wizard configured to guide a user through the configuration steps. The user is thus guided through a series of choices to select a suitable configuration. The output is a configuration file usable by a converting means of the first information system of the IT service provider.

According to a further aspect of the invention, data from an electronic health record of the first information system of the IT service provider and/or other data sources is converted into the second data format used by the healthcare provider. Thus the data stored in the electronic health record can be converted into any data format and transmitted by any data transfer protocol the converter is capable of generating.

According to a further aspect of the invention, a scheduling application of the first information system of the IT service provider is configured to poll data from the electronic health record, to convert and/or transfer said data to the second information system of a healthcare provider. This advantageously enables automation of data transfer at regular intervals from the first information system of the IT service provider to the second information system of the healthcare provider.

Additionally or alternatively, this advantageously enables an event triggered data transfer. e.g. trigger data transfer, if an implant sends new data to the first information system, or if the second information system sends a trigger to the first information system.

According to a further aspect of the invention, the web server, a converting means and/or a data transfer means are implemented as a cloud service. This alternative embodiment provides additional flexibility regarding geographical placement, availability and scalability of the system components.

According to a further aspect of the invention, data stored in the electronic health record is sent to the cloud service for conversion to the second data format used by the healthcare provider. The electronic health record data can thus be kept within the infrastructure of the IT service provider, whereas computing tasks such as converting and exporting the data can be performed in the cloud environment.

According to a further aspect of the invention, data is transferable from the first information system of the IT service provider to the second information system of the healthcare provider and vice versa from the second information system of the healthcare provider to the first information system of the IT service provider.

This bidirectional data transfer capability advantageously provides the possibility of an enhanced data exchange between the first information system of the IT service provider to the second information system of the healthcare provider.

The herein described features of the computer-implemented method for transferring data are also disclosed for the medical data system and vice versa.

For a more complete understanding of the present invention and advantages thereof, reference is now made to the following description taken in conjunction with the accompanying drawings. The invention is explained in more detail below using exemplary embodiments, which are specified in the schematic figures of the drawings, in which:

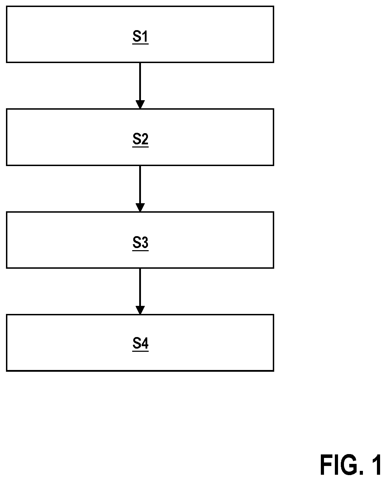

- Fig. 1: shows a flowchart of a computer-implemented method for transferring data, in particular clinical and/or administrative data, from a first information system of an IT service provider to a second information system of a healthcare provider, in particular a hospital, via a data transfer protocol used by the healthcare provider and having a data format used by the healthcare provider according to a preferred embodiment of the invention; and

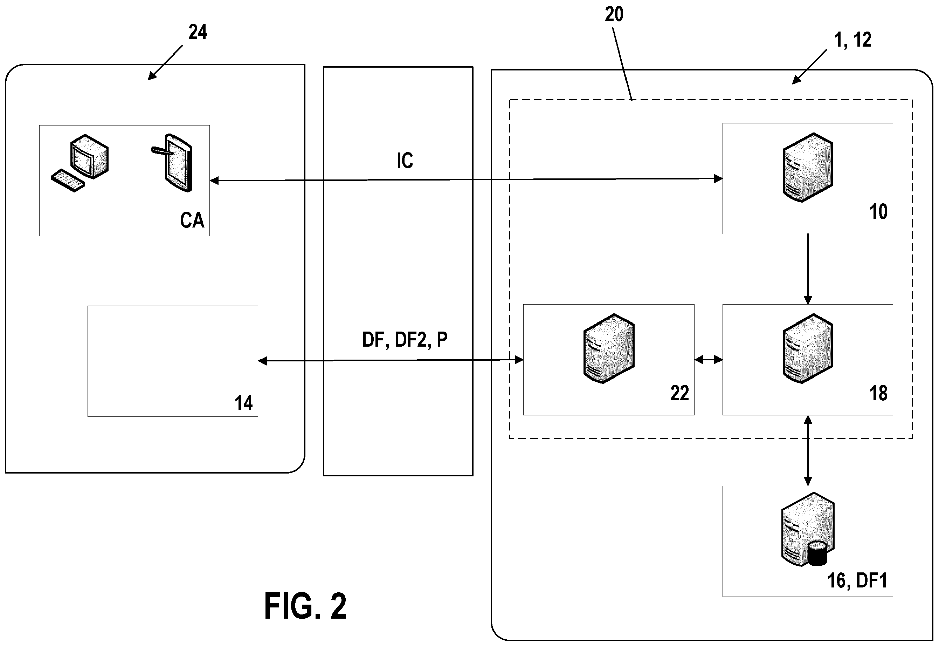

- Fig. 2: shows a diagram of a medical data system for transferring data, in particular clinical and/or administrative data, to a further medical data system of a healthcare provider, in particular a hospital, via a data transfer protocol used by the healthcare provider and having a data format used by the healthcare provider according to the preferred embodiment of the invention.

The computer-implemented method of Fig. 1 for comprises accessing S1 a web server 10 of the first information system 12 of the IT service provider by means of a client application CA, in particular of the healthcare provider, via an internet connection IC.

The method further comprises configuring S2 a data transfer protocol P and a data format DF for the data transfer between the first information system 12 of an IT service provider and the second information system 14 of a healthcare provider on the web server 10 by means of the client application CA, wherein the configured data transfer protocol P and data format DF correspond to the data transfer protocol P and data format DF used by the healthcare provider.

Moreover, the method comprises converting S3 the data of the first information system 12 of an IT service provider, said data having a first data format DF1, into data having a second data format DF2 used by the healthcare provider, and transferring S4 the converted data having the second data format DF2 via the configured data transfer protocol P to the second information system 14 of the healthcare provider.

The client application CA is configured to import a data definition and/or a sample data set from the second information system 14 of the healthcare provider in a structured machine-readable format, in particular JSON, XML, CVS and/or JAML

Furthermore, a parsing algorithm is configured to parse the imported data definition and/or sample data set and suggest a data format DF and/or a data mapping between the first information system 12 of the IT service provider and the second information system 14 of the healthcare provider.

The data of the first information system 12 of the IT service provider having a first data format DF1 is subsequently converted into the second data format DF2 used by the healthcare provider according to the configuration made by the client application CA on the web server 10 of the first information system 12. Alternatively, the client application CA may also be a mobile application with connection to the web server 10. This mobile application would run on a mobile device (e. g. cell phone or tablet) natively.

The client application CA is further configured to provide a configuration wizard configured to guide a user through the configuration steps. In addition, data from an electronic health record 16 of the first information system 12 of the IT service provider and/or other data sources is converted into the second data format DF2 used by the healthcare provider.

A scheduling application of the first information system 12 of the IT service provider is configured to poll data from the electronic health record 16, to convert and/or transfer said data to the second information system 14 of a healthcare provider. Moreover, the web server 10, a converting means 18 and/or a data transfer means 22 are implemented to be arranged within the premises of the IT service provider. Alternatively, the web server 10, the converting means 18 and/or the data transfer means 22 can be implemented as a cloud service 20. In this case, data stored in the electronic health record 16 is sent to the cloud service 20 for conversion to the second data format DF2 used by the healthcare provider.

Data is furthermore transferable from the first information system 12 of the IT service provider to the second information system 14 of the healthcare provider and vice versa from the second information system 14 of the healthcare provider to the first information system 12 of the IT service provider.

Fig. 2 shows a diagram of a medical data system 1 for transferring data, in particular clinical and/or administrative data, to a further medical data system 24 of a healthcare provider, in particular a hospital, via a data transfer protocol P used by the healthcare provider and having a data format DF used by the healthcare provider according to the preferred embodiment of the invention.

The medical data system 1 comprises a web server 10 and a client application CA for accessing the web server 10 via an internet connection IC, wherein the client application CA is adapted to configure a data transfer protocol P and a data format DF for the data transfer between the medical data system 1, in particular of an IT service provider, and the further medical system of the healthcare provider on the web server 10, wherein the configured data transfer protocol P and data format DF correspond to the data transfer protocol P and data format DF used by the healthcare provider.

A converting means 18 is configured to convert data of the medical data system 1, said data having a first data format DF1, into data having a second data format DF2 used by the healthcare provider. In addition, data transfer means 22 are configured to transfer the converted data having the second data format DF2 via the configured data transfer protocol P to the further medical data system 24 of the healthcare provider.

The medical data system 1 comprises or is connected to an electronic health record 16 configured to store medical data and to provide said data to the converting means 18 and/or data transfer means 22.

In addition, the data transfer means 22 of the medical data system 1 is configured to transfer data to the further medical data system 24 and to receive data from the further medical system, wherein said received data is convertible by the converting means 18 into a first data format DF1 used by the medical data system 1 of the IT service provider.

### Reference Signs

- 1: medical data system
- 10: web server
- 12: first information system
- 14: second information system
- 16: electronic health record
- 18: converting means
- 20: cloud service
- 22: data transfer means
- 24: further medical data system
- CA: client application
- D: data
- DF: data format
- DF1: first data format
- DF2: second data format
- IC: internet connection
- P: data transfer protocol
- S1 - S4: method steps

## Claims

1. Computer-implemented method for transferring data (D), in particular clinical and/or administrative data, from a first information system (12) of an IT service provider to a second information system (14) of a healthcare provider, in particular a hospital, via a data transfer protocol (P) used by the healthcare provider and having a data format (DF) used by the healthcare provider, comprising the steps of:
accessing (S1) a web server (10) of the first information system (12) of the IT service provider by means of a client application (CA), in particular of the healthcare provider, via an internet connection (IC);
configuring (S2) a data transfer protocol (P) and a data format (DF) for the data transfer between the first information system (12) of an IT service provider and the second information system (14) of a healthcare provider on the web server (10) by means of the client application (CA), wherein the configured data transfer protocol (P) and data format (DF) correspond to the data transfer protocol (P) and data format (DF) used by the healthcare provider;
converting (S3) the data of the first information system (12) of an IT service provider, said data having a first data format (DF1), into data having a second data format (DF2) used by the healthcare provider; and
transferring (S4) the converted data having the second data format (DF2) via the configured data transfer protocol (P) to the second information system (14) of the healthcare provider.

2. Computer-implemented method of claim 1, wherein the client application (CA) is configured to import a data definition and/or a sample data set from the second information system (14) of the healthcare provider in a structured machine-readable format, in particular JSON, XML, CVS and/or JAML.

3. Computer-implemented method of claim 1 or 2, wherein a parsing algorithm is configured to parse the imported data definition and/or sample data set and suggest a data format (DF) and/or a data mapping between the first information system (12) of the IT service provider and the second information system (14) of the healthcare provider.

4. Computer-implemented method of any one of the preceding claims, wherein the data of the first information system (12) of the IT service provider having a first data format (DF1) is converted into the second data format (DF2) used by the healthcare provider according to the configuration made by the client application (CA) on the web server (10) of the first information system (12).

5. Computer-implemented method of any one of the preceding claims, wherein the client application (CA) is configured to provide a configuration wizard configured to guide a user through configuration steps.

6. Computer-implemented method of any one of the preceding claims, wherein data from an electronic health record (16) of the first information system (12) of the IT service provider and/or other data sources is converted into the second data format (DF2) used by the healthcare provider.

7. Computer-implemented method of claim 6, wherein a scheduling application of the first information system (12) of the IT service provider is configured to poll data from the electronic health record (16), to convert and/or transfer said data to the second information system (14) of a healthcare provider.

8. Computer-implemented method of claim 6 or 7, wherein the web server (10), a converting means (18) and/or a data transfer means (22) are implemented as a cloud service (20).

9. Computer-implemented method of claim 8, wherein data stored in the electronic health record (16) is sent to the cloud service (20) for conversion to the second data format (DF2) used by the healthcare provider.

10. Computer-implemented method of any one of the preceding claims, wherein data is transferable from the first information system (12) of the IT service provider to the second information system (14) of the healthcare provider and vice versa from the second information system (14) of the healthcare provider to the first information system (12) of the IT service provider.

11. Medical data system (1) for transferring data, in particular clinical and/or administrative data, to a further medical data system (24) of a healthcare provider, in particular a hospital, via a data transfer protocol (P) used by the healthcare provider and having a data format (DF) used by the healthcare provider, comprising:
a web server (10) and a client application (CA) for accessing the web server (10) via an internet connection (IC), wherein the client application (CA) is adapted to configure a data transfer protocol (P) and a data format (DF) for the data transfer between the medical data system (1), in particular of an IT service provider, and the further medical system of the healthcare provider on the web server (10), wherein the configured data transfer protocol (P) and data format (DF) correspond to the data transfer protocol (P) and data format (DF) used by the healthcare provider;
a converting means (18) configured to convert data of the medical data system (1), said data having a first data format (DF1), into data having a second data format (DF2) used by the healthcare provider; and
a data transfer means (22) configured to transfer the converted data having the second data format (DF2) via the configured data transfer protocol (P) to the further medical data system (24) of the healthcare provider.

12. Medical data system of claim 10, wherein the medical data system (1) comprises or is connected to an electronic health record (16) configured to store medical data and to provide said data to the converting means (18) and/or data transfer means (22).

13. Medical data system of claim 10 or 11, wherein the data transfer means (22) of the medical data system (1) is configured to transfer data to the further medical data system (24) and to receive data from the further medical data system (24), wherein said received data is convertible by the converting means (18) into a first data format (DF 1) used by the medical data system (1) of the IT service provider.

14. Computer program with program code to perform the method of any one of claims 1 to 10 when the computer program is executed on a computer.

15. Computer-readable data carrier containing program code of a computer program for performing the method of any one of claims 1 to 10 when the computer program is executed on a computer.
